(19) Europäisches Patentamt
European Patent Office
Office européen des brevets

(11) Numéro de publication: **0 280 597 A1**

(12) # DEMANDE DE BREVET EUROPEEN

(21) Numéro de dépôt: 88400206.4

(22) Date de dépôt: 29.01.88

(51) Int. Cl.4: **C 07 B 59/00**
C 01 B 15/01, C 01 B 15/023

(30) Priorité: 03.02.87 FR 8701282

(43) Date de publication de la demande:
31.08.88 Bulletin 88/35

(84) Etats contractants désignés:
BE CH DE FR GB IT LI NL SE

(71) Demandeur: ATOCHEM
4 & 8, Cours Michelet La Défense 10
F-92800 Puteaux (FR)

(72) Inventeur: Schirmann, Jean-Pierre
49 Chemin de la Glacière
F-69600 Oullins (FR)

Barieux, Jean-Jacques
3 Rue Mozart
F-69100 Villeurbanne (FR)

(74) Mandataire: Rochet, Michel et al
ATOCHEM Département Propriété Industrielle La Défense 10 4 & 8 Cours Michelet
F-92800 Puteaux (FR)

(54) **Composés peroxydés renfermant l'isotope 17o.**

(57) L'invention concerne des composés peroxydés contenant l'isotope $^{17}O$.

Elle s'applique tout particulièrement aux peroxydes d'hydrogène contenant $^{17}O$, aux peroxydes et hydroperoxydes préparés à partir de $H_2{}^{17}O_2$ et à toute une famille de molécules marquées en dérivant.

Ces molécules, non radioactives, intéressent notamment les domaines de la médecine et de la biologie.

EP 0 280 597 A1

**Description**

COMPOSES PEROXYDES RENFERMANT L'ISOTOPE $^{17}O$

La présente invention concerne une nouvelle famille de composés peroxydés. Elle a également pour objet un procédé de préparation de tels composés ainsi que les applications de ces composés en tant que molécules marquées aussi bien qu'en tant qu'intermédiaires de synthèse de molécules marquées.

Les composés peroxydés conformes à l'invention sont caractérisés en ce que tout ou partie des atomes d'oxygène formant le groupement -O-O- est constitué de l'isotope $^{17}O$.

Parmi ces composés l'invention concerne plus particulièrement les composés dans lesquels plus 0,1 % en poids de l'oxygène peroxydique -O-O- provient de l'isotope $^{17}O$ cette proportion étant de préférence supérieure à 1 % et inférieure de 80 %. Lorsque l'isotope $^{17}O$ est associé à $^{16}O$ et pour des raisons de simplification, ce groupement peroxydique caractéristique des composés conformes à l'invention s'écrira présentement $-^{16}O-^{17}O-$, étant bien entendu que cette écriture ne saurait limiter l'invention à une répartition $^{16}O/^{17}O$ en parts égales, seules ne pouvant être retenues que les indications données précédemment concernant la part représentée par $^{17}O$ dans le groupement -O-O-.

Les composés peroxydés conformes à l'invention peuvent être représentés par les formules

$R - ^{16}O - ^{17}O - R'$ (I)ou

$R - ^{17}O - ^{17}O - R'$ (II)

dans lesquelles R et R' qui peuvent être identiques ou différents peuvent représenter :

- un atome d'hydrogène
- un radical $R_1$ alkyle, linéaire ou ramifié, alcényle, alcynyle, cycloalkyle, alkoxy ou cycloalkoxy, aryle, ou un groupement minéral, organométallique ou organométalloïdique, ces derniers comprenant au moins l'un des dits radicaux $R_1$ et un métal ou métalloïde choisi dans le groupe constitué par Al, Ge, Pb, P, Li, Sn, Sb, Te, Se, As, B, ledit groupement organométallique ou métalloïdique pouvant lui-même comporter un ou plusieurs groupements $-^{16}O-^{17}O-$
- l'un des groupements

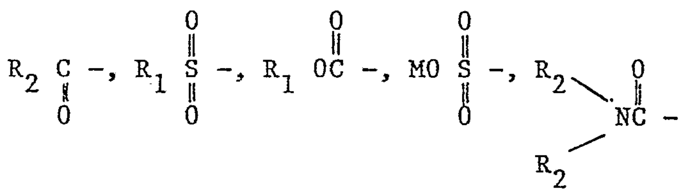

dans lesquels les groupements $R_2$ peuvent avoir l'une des significations données ci-avant pour le radical $R_1$ et M représente un atome d'hydrogène ou un métal alcalin, les dits radicaux $R_1$ ou $R_2$ qui peuvent renfermer jusqu'à 20 atomes de carbone pouvant être substitués par un ou plusieurs atomes de fluor, chlore, brome ou groupement(s) OH ou $NO_2$

-R et R' pouvant représenter ensemble un atome ou groupement divalent T, le dit groupement divalent T pouvant lui-même être purement hydrocarboné ou contenir un ou plusieurs enchaînements $-^{16}O-^{17}O-$, les valences libres du dit enchaînement pouvant être liées à des groupements organiques ou inorganiques.

A titre d'illustration des atomes ou groupements divalents T on peut citer notamment les atomes de calcium, magnésium, baryum, zinc, cadmium, les groupements $-CH(CH_3)-O-CH(CH_3)-$, $-CH_2-O-CH_2-$, $-B(OH)_2-{}^{16}O-{}^{17}O-B(OH)_2-$, $-C(CH_3)_2-{}^{16}O-{}^{17}O-C(CH_3)_2-$, $-C(CH_3)(C_2H_5)-{}^{16}O-{}^{17}O-C(CH_3)(C_2H_5)-$,

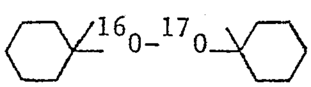 ,

$- C(CH_3)_2 -{}^{16}O-{}^{17}O- C(CH_3)_2 -{}^{16}O-{}^{17}O- C(CH_3)_2 -$

Il ressort de la signification des différents symboles que l'invention concerne en premier lieu le peroxyde d'hydrogène renfermant l'isotope $^{17}O$, ainsi que les hydroperoxydes, peroxydes, peracides, peresters et plus généralement l'ensemble des composés peroxydés et notamment les composés peroxydés pouvant être préparés à partir du peroxyde d'hydrogène.

L'invention concerne tout particulièrement les composés de formules (I) ou (II) dans lesquelles l'un au moins des symboles R et R' -l'autre pouvant en outre représenter l'hydrogène - représente un radical méthyle, éthyle, isopropyle, n-butyle, t-butyle, t-amyle, cyclohexyle, méthoxy, éthynyle, propyne-2yle, p-diisopropylphényle, méthyl-1 cyclohexyle, tétrahydro 1,2,3,4, naphtyl-1, acétyle, propio nyle, isobutyryle, pivaloyle, stéaroyle, acryloyle, oléoyle, benzyloyle, nonanoyle, palmitoyle, chloracétyle, trichloracétyle, trifluoracétyle, chloro-4 benzoyle.

A titre d'illustration des composés peroxydés conformes à l'invention, on mentionnera notamment les composés suivants renfermant $-^{17}O$-dans le groupement péroxyde :

- les hydroperoxydes de méthyle, éthyle, isopropyle, n-,sec- et t-butyle, de t-amyle, de cyclohexyle, de tétraméthyl-1,1,3,3, butyle, de méthoxy-1 cyclohexyle, de stéaryle

- les hydroxyhydroperoxydes de formule $R_3CH(OOH)(OH)$ dans laquelle $R_3$ représente H, $CH_3$, $CCl_3$, n-$C_4H_9$, n-$C_7H_{15}$, n-$C_8H_{17}$,
- les hydroperoxydes de formule $[R_4H(OH)CO]_2$ dans laquelle $R_4$ représente H, $CH_3$, $CCl_3$, n-$C_4H_9$, n-$C_7H_{15}$, n-$C_8H_{17}$,
- le chloro-2 hydropéroxy-1 cyclohexanol
- le dioxy-1,1 bis cyclohexanol
- le tétraméthyl-3,3,6,6 tétroxane-1,2,4,5
- l'hexaméthyl-3,3,6,6,9,9 hexoxanane-1,2,4,5,7,8
- le trioxolane-1,2,4
- le diméthyl-3,5 trioxalane-1,2,4
- le diphényl-3,5 trioxalane-1,2,4
- les peroxydes de diméthyle, perfluorodiméthyle, diéthyle, t. butylméthyle, diisopropyle, perfluoro-di t-butyle, dicumyle
- le peroxyborate de sodium, tétrahydrate ou monohydrate
- le dioxybis (triéthylstannane)
- les acides performique, peracétique, peroxypropionique, peroxybutyrique, perbenzoïque, m-chloroperbenzoïque, diperoxyphtalique, diperoxyhexanoïque, trifluoroperacétique, trichloroperacétique
- les peroxydes de diacétyle, de bis (chloroacétyle), d'acétylbenzoyle, de dioctanoyle, de bis (phénylacétyle), bis (trichloroacétyle), bis (trifluoracétyle).
- les peroxydicarbonates de diéthyle, de diisopropyle, de dicyclohexyle
- les peroxydes de bis (méthylsulfonyle), d'acétyle t-butyl sulfonyle, d'acétyle cyclohexylsulfonyle, de bis (benzène sulfonyle)
- le peracétate de t-cumyle, le perbenzoate de t-butyle, le diperoxyadipate de di-t.butyle.
- le diisopropyl peroxydicarbonate, le t-butyl peroxypivalate.

L'invention a également pour objet un procédé de préparation des composés peroxydés précités, procédé mettant en oeuvre, en tant que représentant unique ou partiel de l'oxygène, l'isotope $^{17}O$. Autrement dit, on utilise dans l'invention soit le seul $^{17}O$, soit de l'oxygène $^{16}O$ enrichi en $^{17}O$.

L'isotope $^{17}O$ existe dans la nature à très faible concentration (0,04 %) et l'isolement de cet isotope $^{17}O$ ou l'enrichissement de $^{16}O$ en $^{17}O$ ne constituent pas en eux-mêmes des objets de l'invention. De telles techniques, qui comprennent par exemple la distillation fractionnée ou l'électrolyse d'eau lourde ou la séparation par laser, sont décrits dans la littérature.

Le premier composé peroxydé conforme à l'invention (peroxyde d'hydrogène) peut être très avantageusement préparé en utilisant le procédé d'oxydo-réduction des quinones. Selon ce procédé la quinone et plus précisément l'anthraquinone ou les alkylanthraquinones est hydrogénée, en général en présence d'un catalyseur tel que le nickel Raney, ou le palladium sur alumine activée, puis oxydée.

Dans le procédé conforme à l'invention, il convient soit d'utiliser l'oxygène $^{17}O$ ou l'oxygène $^{16}O$ enrichi en $^{17}O$ en remplacement de l'air, soit d'utiliser de l'air enrichi en oxygène $^{17}O$. La réaction d'hydrogénation s'effectue généralement en phase solvant, ces solvants pouvant par exemple consister en esters des acides acétique, propionique, adipique, succinique, sébacique, éventuellement mélangés à des alcools, en mélanges d'alcools et d'acides dicarboxyliques, en mélanges d'alcools et de naphtalène substitué.

La solution résultant de cette hydrogénation est oxydée comme indiqué précédemment puis lavée pour en extraire une solution aqueuse de peroxyde d'hydrogène $H^{16}O^{17}OH$ et/ou $H_2{}^{17}O_2$.

Le peroxyde d'hydrogène conforme à l'invention présente l'ensemble des propriétés physiques et chimiques du peroxyde d'hydrogène renfermant $^{16}O$. Il présente en outre un spectre de résonance magnétique caractérisé par un déplacement chimique de $\delta = 180$ ppm par rapport à $H_2{}^{17}O$ pris comme référence. Son spectre de masse indique clairement la présence de l'ion m/e des pics parents de 36 et 35.

Les appareils de résonance magnétique nucléaire modernes permettent d'identifier l'oxygène $^{17}O$ et de doser le taux d'enrichissement.

Les autres composés peroxydés en objet de la présente invention peuvent, d'une manière générale être préparés en suivant les techniques connues, pour autant que l'isotope $^{17}O$ ait été utilisé à un moment quelconque de la préparation des dits composés peroxydés.

Cependant, une technique particulièrement recommandée consiste à préparer ces composés peroxydés à partir du peroxyde d'hydrogène $H_2{}^{17}O_2$ ou H $^{16}O^{17}O$ H décrit ci-avant, seul ou associé à $H_2{}^{16}O_2$.

Les hydroperoxydes d'alkyle peuvent être préparés à partir des halogénures d'alkyle ou des alcools tertiaires par réaction avec $H_2{}^{17}O_2$ ou $H^{16}O^{17}OH$ en milieu acide. On peut également les préparer par oxydation directe des hydrocarbures correspondants par $^{17}O_2$ ou -$^{17}O$-$^{16}O$-

Les hydroxy ou dihydroxyhydroperoxydes ou peroxydes peuvent être obtenus à partir de $H_2{}^{17}O_2$ ou H $^{16}O^{17}O$ H et d'aldéhydes ou de cétones.

Les peroxydes de dialkyle peuvent être obtenus à partir de $H_2{}^{17}O_2$ ou H $^{16}O^{17}O$ H et du réactif approprié qui, selon les conditions de la réaction et notamment l'acidité ou la basicité du milieu, peut consister en sulfate de dialkyle, sulfonate de dialkyle, halogénures d'alkyles, alcools, glycols, éthers, esters, amines, amides ou encore en composés portant une double liaison oléfinique, tels que les hydrocarbures oléfiniques acycliques et cycliques, les éthers vinyliques et isopropényliques, les enamines, N-vinylamides, vinylsulfonates, sulfones divinyliques ou composés $\alpha$, $\beta$ insaturés tels que l'acrylate de méthyle, l'acrylamide, l'acrylonitrile.

Les peracides peuvent être obtenues par la réaction équilibrée de peroxyde d'hydrogène avec un acide

carboxylique, réaction à catalyse acide.

Les peroxydes de diacyle peuvent être préparés par réaction en présence d'une base d'un chlorure d'acyle ou d'un anhydride d'acide avec le peroxyde d'hydrogène, le peroxyde de sodium ou encore avec un peracide.

Les peresters peuvent être préparés par réaction d'un hydroperoxyde d'alkyle avec un agent d'acylation, tel que chlorure d'acyle, anhydride, cétène, chlorure de sulfonyle, phosgène, chloroformate d'alkyle, isocyanate, chlorure de carbamoyle, acide carboxylique et ester.

Les indications qui précèdent concernent essentiellement des moyens d'accès aux différents composés peroxydés conformes à l'invention au départ de peroxyde d'hydrogène. Bien évidemment celle-ci ne saurait être limitée à de telles voies d'accès et s'étend en fait à tout mode de préparation des composés de formule I, c'est-à-dire aux composés peroxydés dans la formule desquels intervient l'isotope $^{17}O$.

Les composés peroxydés conformes à l'invention constituent en eux-mêmes des molécules tracées ou marquées du fait de la présence de l'isotope $^{17}O$. De ce fait ils peuvent être utilisés pour l'étude de nombreuses réactions et notamment des réactions d'oxydation comportant le transfert de l'atome d'oxygène sur l'agent réducteur, in vivo ou in vitro.

Ces composés peuvent aussi, du fait de leur nature peroxydique, servir à la préparation d'un grand nombre de molécules marquées. Parmi ces molécules qui constituent également un autre objet de l'invention, on citera notamment, à partir d'hydroperoxydes ou de peroxydes

- les alcools obtenus par réduction des hydroperoxydes d'alkyle,
- les acides insaturés et esters, à partir d'aldéhydes et des composés peroxydés,
- les hydroxylamines, imines et nitrones à partir d'amines et des composés peroxydés,
- les acides carboxyliques, à partir de cétones et des composés peroxydés,
- les phosphates à partir de phosphines et des composés peroxydés,
- les oxydes de phosphines à partir de phosphines et des composés peroxydés,
- les sulfoxydes à partir de sulfures et de composés peroxydés,
- les alcools et phénols à partir des réactifs de Grignard et de composés peroxydés,
- les époxydes à partir d'oléfines et de composés peroxydés,
- les phénols à partir de composés aromatiques et de composés peroxydés,
- les amides à partir des nitriles,
- les cétones à partir d'alcools secondaires et de composés peroxydés,
- les quinones à partir de phénols et de composés peroxydés.

Parmi les familles de produits précités, on citera à titre purement illustratif, les composés suivants, le méthanol, l'éthanol, le propanol, l'isopropanol, l'alcool benzylique, l'acétone, la méthyléthycétone, la cyclohexanone, l'oxyde de mesityle, le diacétonealcool, l'acétophénone, la benzophénone, le formol, l'acétaldéhyde, le propionaldéhyde, les butyraldéhydes, l'acroléine, le crotonaldéhyde, l'acide formique, l'acide acétique, l'acide propionique, les acides butyriques, les acides aminés, l'acide acrylique, l'acide méthacrylique, l'acide benzoïque, l'acide paranitrobenzoïque, l'acide glutarique, l'acide oxalique, l'acide succinique, l'acide adipique, les acides phtaliques, l'anhydride acétique, l'anhydride maléique, l'anhydride phtalique, le phénol, le pyrocatéchol, l'hydroquinone, la naphtoquinone, l'anthraquinone, l'éthyl-2 anthraquinone, l'acétate de méthyle, l'acétate d'éthyle, la butyrolactone, la caprolactone, le formamide, l'acétamide, le benzamide, etc ...

Les molécules marquées précitées sont susceptibles d'un grand nombre d'applications, notamment dans les domaines de la médecine et de la biologie, où la présence de l'isotope $^{17}O$ permet, du fait de sa non-radioactivité, d'établir des schémas de métabolisme.

Les exemples suivants illustrent l'invention.

EXEMPLE 1

On hydrogène sur un catalyseur constitué par du palladium déposé sur silicoaluminate à raison de 2 % en métal précieux, une solution synthétique contenant 66,8 g/litre de tétrahydro-5,6,7,8 éthyl-2 anthraquinone et 6,7 g/litre d'éthyl-2 anthraquinone, soit 73,5 g/litre de soluté total dans un solvant constitué par 57 % en volume d'acétate de méthylcyclohexyle et 43 % en volume d'une coupe d'hydrocarbures aromatiques en $C_9$. L'hydrogénation est conduite à 70° C. Lorsque celle-ci est terminée, on purge soigneusement la phase gazeuse du réacteur à l'aide d'azote. La phase hydrogénée maintenue sous azote est séparée du catalyseur et une part aliquote de 200 g de solution est introduite dans un réacteur de 500 ml et portée à 70° C. On introduit alors sous agitation et progressivement 0,9 litre d'oxygène enrichi ($^{17}O$ = 10 %). Lorsque l'absorption d'oxygène cesse, le mélange réactionnel obtenu est soumis à une extraction par 100 ml d'eau bipermutée deux fois de suite. On réunit les deux extraits aqueux et on dose par iodométrie 0,03 mole de peroxyde d'hydrogène, ce qui correspond à un rendement de 80 %.

L'analyse par RMN $^{17}O$ fournit un spectre qui montre un pic de résonance ayant un déplacement chimique de $\delta$ = 180 ppm dans l'eau, $H_2{}^{17}O$ (commercial) servant de référence interne. L'identification est confirmée par la spectrométrie de masse qui donne deux pics parents de masse respective 35 et 36 correspondant à $H\text{-}^{17}O\text{-}^{17}O\text{-}H$ et $H\text{-}^{16}O\text{-}^{17}O\text{-}H$.

EXEMPLE 2

Dans un réacteur de 500 ml, contenant 300 ml d'éther anhydre, muni d'une agitation et refroidi à - 70° C, on ajoute simultanément et lentement en 75 minutes 0,9 litre d'oxygène enrichi ($^{17}O$ ~ 10 %) et 70 ml d'une

solution étherée (0,53 molaire) de chlorure de tertiobutyle magnésium. On laisse remonter la température lentement à 0°C, puis on procède à une hydrolyse lente par addition goutte à goutte de 50 ml d'acide chlorhydrique dilué (0,72 N). Après décantation et extraction de la phase aqueuse par 100 ml d'éther, on rassemble les phase étherées et on ajuste leur volume à 500 ml. Le dosage par iodométrie permet de constater qu'il s'est formé 2,72 g d'hydroperoxyde de terbutyle, ce qui correspond à un rendement de 86 % par rapport au magnésium et de 80 % par rapport à l'oxygène engagé. L'analyse par RMN $^{17}O$ fournit les résultats suivants, en prenant $H_2{}^{17}O$ comme référence interne :

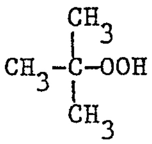

95 %  $\delta\ ^{17}O$ = 208 et 243 ppm

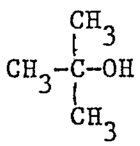

5 %  $\delta\ ^{17}O$ = 64 ppm

EXEMPLES 3 à 5

A 2 $cm^3$ de solution aqueuse de peroxyde d'hydrogène marqué $^{17}O$ préparé selon l'exemple 1, on ajoute à température ambiante 3 $cm^3$ d'un acide carboxylique : acétique, propionique, trifluoracétique ainsi qu'une trace d'acide sulfurique. On laisse réagir durant 2 heures et on examine le mélange réactionnel par RMN $^{17}O$. On observe la formation des acides percaboxyliques marqués correspondants. Les acides carboxyliques correspondants marqués peuvent être obtenus de la même façon avec $H_2{}^{17}O$. Les déplacements chimiques sont les suivants en prenant $H_2{}^{17}O$ comme référence interne :

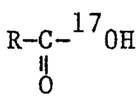 (*)

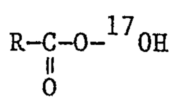 (**)

| | | |
|---|---|---|
| $CH_3-COOH$ | $\delta$ = 255 | $\delta$ = 273 |
| $C_2H_5-COOH$ | $\delta$ = 254 | $\delta$ = 266 et 280 |
| $CF_3-COOH$ | $\delta$ = 245 | $\delta$ = 260 et 280 |

(* les 2 O sont équivalents et donnent une raie unique)
(** les données correspondent aux oxygènes peroxydiques ; le $>C = O$ se trouve vers $\delta$ 332)

EXEMPLE 6

On fait réagir 10 $cm^3$ de solution étherée obtenue selon l'exemple 2 avec 10 $cm^3$ d'acide sulfurique 10 %. Par RNM $^{17}O$ on observe la formation de peroxyde de diterbutyle $\delta^{17}O$ = 269 ppm (référence interne $H_2{}^{17}O$).

EXEMPLE 7

A 2 $cm^3$ de solution aqueuse de peroxyde d'hydrogène marqué à l'oxygène 17 preparé selon l'exemple 1 on ajoute 10 $cm^3$ d'acide sulfurique 10 0/0 et 2 $cm^3$ de terbutanol, à température ordinaire. Par RMN $^{17}O$ on observe la formation de peroxyde de diterbutyle caractérisé par un déplacement chimique $\delta^{17}O$ = 269 ppm (référence interne $H_2{}^{17}O$).

**Revendications**

1. A titre de produits industriels nouveaux, des composés peroxydés caractérisés en ce que tout ou partie des atomes d'oxygène formant le groupement -O-O- est constitué de l'isotope $^{17}O$.

2. Composés selon la revendication 1, caractérisés en ce qu'ils renferment plus de 0,1 % en poids d'oxygène $^{17}O$ dans le groupement -O-O-.

3. Composés selon la revendication 1, caractérisés en ce qu'ils renferment de 1 à 80 % en poids d'oxygène $^{17}O$ dans le groupement -O-O-.

4. Composés selon l'une quelconque des revendications 1 à 3, caractérisés en ce qu'ils répondent à l'une des formules :

$R - {}^{16}O - {}^{17}O - R'$ (I) ou

$R - {}^{17}O - {}^{17}O - R'$ (II)

dans lesquelles R et R' qui peuvent être identiques ou différents peuvent représenter :

- un atome d'hydrogène

- un radical $R_1$ alkyle, linéaire ou ramifié, alcényle, alcynyle, cycloalkyle, alkoxy ou cycloalkoxy, aryle, ou un groupement minéral, organométallique ou organométalloïdique, ces derniers comprenant au moins l'un des dits radicaux $R_1$ et un métal ou métalloïde choisi dans le groupe constitué par Al, Ge, Pb, P, Li, Sn, Sb, Te, Se, As, B, le dit groupement organométallique ou métalloïdique pouvant lui-même comporter un ou plusieurs groupements $-{}^{16}O-{}^{17}O-$

- l'un des groupements

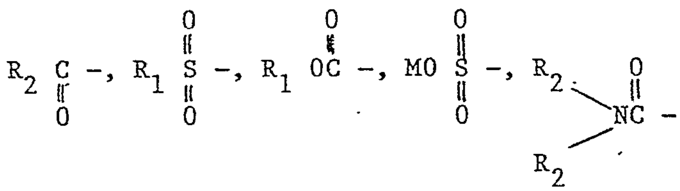

dans lesquels les groupements $R_2$, peuvent avoir l'une des significations données ci-avant pour le radical $R_1$ et M représente un atome d'hydrogène ou un métal alcalin, les dits radicaux $R_1$ ou $R_2$ qui peuvent renfermer jusqu'à 20 atomes de carbone pouvant être substitués par un ou plusieurs atomes de fluor, chlore, brome ou groupement(s) OH ou $NO_2$

-R et R' pouvant représenter ensemble un atome ou groupement divalent T, ledit groupement divalent T pouvant lui-même être purement hydrocarboné ou contenir un ou plusieurs enchaînements $-{}^{16}O-{}^{17}O-$ les valences libres dudit enchaînement pouvant être liées à des groupements organiques ou inorganiques.

5. Composés selon l'une quelconque des revendications 1 à 4, choisi dans le groupe constitué par le peroxyde d'hydrogène, les hydroperoxydes, peroxydes, peracides, et peresters.

6. procédé de préparation de peroxyde d'hydrogène de formule $H_2{}^{17}O_2$ ou $H^{17}O^{16}OH$, par oxydo-réduction de quinone caractérisé en ce que l'oxydation est réalisée en utilisant l'oxygène $^{17}O$ ou l'oxygène $^{16}O$ enrichi en oxygène $^{17}O$ ou l'air enrichi en oxygène $^{17}O$.

7. Procédé selon la revendication 6, caractérisé en ce que la quinone est choisie dans le groupe constitué par l'anthraquinone et les alkylanthraquinones.

8. Procédé de préparation des hydroperoxydes, peroxydes, peracides et peresters selon l'une quelconque des revendications 1 à 5, caractérisé en ce que le réactif peroxydé de départ est constitué en tout ou partie par $H_2{}^{17}O_2$ ou $H^{17}O^{16}OH$.

Office européen des brevets

# RAPPORT DE RECHERCHE EUROPEENNE

Numero de la demande

EP 88 40 0206

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | TETRAHEDRON LETTERS, vol. 26, no. 17, 1985, pages 2051-2054, Pergamon Press Ltd, Oxford, GB; A.L. BAUMSTARK et al.: "17O-Enriched alpha-azohyddroperoxides: 17O NMR spectroscopy, 17O-labeling reagents"<br>* En entier * | 1-3,5 | C 07 B 59/00<br>C 01 B 15/01<br>C 01 B 15/023 |
| X | CHEMICAL ABSTRACTS, vol. 103, no. 20, 18 novembre 1985, pages 711-712, résumé no. 170875b, Columbus, Ohio, US; R. CURCI et al.: "Oxygen-17 NMR of organic peroxides: chromium(VI) and molybdenum(VI) oxide diperoxide as compared to simple organic peroxides", & J. MOL. CATAL. 1985, 32(2), 251-7<br>* Résumé * | 1-5 | |
| X | HELVETICA CHIMICA ACTA, vol. XLIV, nos. 98-99, fascicule III, 2 mai 1961, pages 865-880, Bâle, CH; H.A. CHRIST et al.: "Chemische Verschiebungen in der kernmagnetischen Resonanz von 17O in organischen Verbindungen 1) 2)"<br>* Pages 865,866, lignes 1-5; page 872, composés nos. 97,98,99 * | 1,5 | |

| DOMAINES TECHNIQUES RECHERCHES (Int. Cl.4) |
|---|
| C 07 B 59/00<br>C 01 B 15/00 |

Le présent rapport a été établi pour toutes les revendications

| Lieu de la recherche | Date d'achèvement de la recherche | Examinateur |
|---|---|---|
| LA HAYE | 16-05-1988 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant